# EUROPEAN PATENT APPLICATION

(11) **EP 4 450 623 A1**
(43) Date of publication of application: **23.10.2024**
(21) Application number: 22906559.4
(22) Date of filing: 13.12.2022
(51) Int. Cl.: C12N 15/113, C12N 15/11, A61K 48/00

(54) **USE OF MIRNA-2911 MOLECULE AS NUCLEIC ACID STABILIZER**

(30) Priority: 14.12.2021 CN 202111523686
(71) Applicant: Lnctac Co., Ltd., Chengdu, Sichuan 610219 (CN)
(72) Inventor: ZHANG, Xiaoxia, Chengdu, Sichuan 610219 (CN); ZENG, Miao, Chengdu, Sichuan 610219 (CN)
(74) Representative: KATZAROV S.A.
(86) International application number: PCT/CN2022/138695
(87) International publication number: WO 2023/109814

(57) **Abstract**

The present invention relates to a use of a miRNA-2911 molecule as a nucleic acid stabilizer. Specifically, the structure of the miRNA-2911 molecule for improving the stability of a target nucleic acid is shown in the following formula: GGX₁X₂GGGGG-(L)ₙ-X₃X₄GGX₅X₆X₇GGGX₈ (SEQ ID NO: 1), wherein X₁, X₂, X₃, X₄, X₅, X₆, X₇, and X₈ are independently selected from A, C, T, and U; n is 0 or 1; and L represents a linker group having a length of no more than 10 nucleotides.

## Description

### FIELD OF THE INVENTION

The disclosure relates to the fields of biomedicine and biotechnology, in particular to the use of a miRNA-2911 molecule as a nucleic acid stabilizer.

### BACKGROUND OF THE INVENTION

By utilizing the ability of nucleic acid to bind to proteins or other targets, a variety of nucleic acid tools or drugs, including ASO drugs, mRNA drugs, RNA apatmers, and regulatory RNAs, can be developed. Among them, mRNA is developed as an mRNA drug based on protein translation. Nucleic acid apatmers can bind to targets (such as proteins, small molecules, cells, etc.), play a role similar to "chemical antibodies", and are applied in the field of nucleic acid drugs. However, these nucleic acid drugs are susceptible to be degraded by nucleases, have unstable affinity with the target, and are difficult to be located and transported.

In order to protect nucleic acid drugs from degradation, a variety of methods have been developed against the nuclease degradation, including both chemical and non-chemical modification methods. Currently, widely used methods are mainly chemical modification methods, including phosphate backbone modification, sugar modification, phosphoric acid modification and base modification, etc., which are used to enhance the stability of nucleic acid drugs. However, chemically modified nucleic acid drugs may have potential safety issues, such as inducing an immune response by activating toll-like receptors, causing cytotoxicity (such as proteinuria caused by inducing apoptosis), impairing coagulation, and decreasing thrombocytopenia, etc.

There are very few non-chemical modification methods reported at present. In 1989, a short-chain oligonucleotide capable of forming an extremely stable secondary structure was reported. In 1993, Coulson's research group found that a thermodynamically stable hairpin (GCGAAAGC), which can protect oligonucleotides from being degraded by the 3'-terminal exonuclease, and optimized the hairpin to obtain a min-hairpin (GCGAAGC). However, the protective effect of this method is very limited. In 2002, U.S. Pat. No. 7022832B2 disclosed that by designing the 5' or/and 3' end of an oligonucleotide as hairpin structures, it can play a protective role in 10% heat-inactivated serum, and achieve gene knock-down in animals with the assistance of nano-materials. However, it has not been widely used due to its limited protective effect. In 2002, U.S. Pat. No. 6121434A disclosed that continuous G bases (wherein the number of G is 0-10) can protect oligonucleotides. However, this method is not widely used because it is usually used in combination with thio-modification. Similarly, mRNA is also susceptible to be degraded by nucleases.

Zhang Chenyu's research group discovered miRNA-2911 in the decoction of honeysuckle, indicating that it has good stability, and found that miRNA-2911 has antiviral activity. However, whether miRNA-2911 can protect nucleic acid from degradation has not been reported so far.

Accordingly, there is an unmet need for substances and methods for improving the stability of nucleic acids and/or protecting nucleic acids from degradation.

### SUMMARY OF THE INVENTION

The present disclosure is based on the finding that miRNA-2911 and a derivative thereof can be used to protect nucleic acids and enhance their stability in serum.

In a first aspect, the present disclosure provides a miRNA-2911 and a nucleic acid molecule derived therefrom for improving the stability of a nucleic acid of interest, characterized in comprising the following sequence:
GGX₁X₂GGGGG-(L)ₙ-X₃-X₄GGX₅X₆X₇GGGX₈ (SEQ ID NO: 1)
wherein:
   X₁, X₂, X₃, X₄, X₅, X₆, X₇ and X₈ are independently selected from A, C, T and U;
   n is 0 or 1;
   L represents a linker group of no more than 10 nucleotides in length.

In some embodiments, the linker group comprises G. In some embodiments, the linker group consists of G(s). In some embodiments, the linker group is 1-5 nucleotides in length. In some embodiments, the linker group is GGGGG (SEQ ID NO: 30).

In some embodiments, the miRNA-2911 and the nucleic acid molecule derived therefrom comprise the following sequence:
GGX₁X₂GGGGGX₃X₄GGX₅X₆X₇GGGX₈ (SEQ ID NO: 3)
wherein:
   X₁, X₂, X₃, X₄, X₅, X₆, X₇ and X₈ are independently selected from A, C, T and U.

In some embodiments, X₁, X₂, X₄, and X₆ are identical. In some embodiments, X₁, X₂, X₄ and X₆ are identical and each are A, C or U. In some embodiments, X₃, X₅, and X₈ are identical. In some embodiments, X₃, X₅ and X₈ are identical and each are A, C or U. In some embodiments, X₁, X₂, X₃, X₄, X₅, X₆, and X₈ are independently selected from A, C, and U, and X₇ is independently selected from A, C, T, and U. In some embodiments, when any one of X₁, X₂, X₃, X₄, X₅, X₆ and X₈ is U, the remaining nucleotides of X₁, X₂, X₃, X₄, X₅, X₆ and X₈ each are U. In some embodiments, X₁, X₂, X₃, X₄, X₅, X₆, X₇, and X₈ each are A, C, or U. In some embodiments, when X₇ is T, X₁, X₂, X₄, and X₆ each are C, and X₃, X₅, and X₈ each are A. In some embodiments, X₁, X₂, X₃, X₄, X₅, X₆, X₇, and X₈ each are A.

In some embodiments, the miRNA-2911 and the nucleic acid molecule derived therefrom comprise or consist of one or more (for example, 2) SEQ ID NO: 1 or SEQ ID NO: 3 directly linked or indirectly linked by a linker. In some embodiments, the linker is no more than 10 nucleotides in length. In some embodiments, the linker comprises G. In some embodiments, the linker consists of G(s). In some embodiments, the linker is 1-5 nucleotides in length. In some embodiments, the linker is GGGGG (SEQ ID NO: 30).

In some embodiments, the miRNA-2911 and the nucleic acid molecule derived therefrom comprise or are selected from the sequences of SEQ ID NO: 4-10.

In some embodiments, the nucleic acid of interest is DNA or RNA. In some embodiments, the nucleic acid of interest is modified or unmodified, e.g., unthiomodified. In some embodiments, the miRNA-2911 and the nucleic acid molecule derived therefrom are DNA or RNA.

In some embodiments, the miRNA-2911 and the nucleic acid molecule derived therefrom are modified or unmodified, e.g., unthiomodified.

In some embodiments, the nucleic acid of interest is 8-5000 nt in length, such as 8-4000 nt, 8-3000 nt, 8-2000 nt, 8-1000 nt, 8-500 nt, 8-200 nt, 8-150 nt, 8-100 nt, 8 -80 nt, 8-50 nt, 8-40 nt, 8-30 nt, 8-20 nt, 8-10 nt, 30-180 nt, 20-100 nt, and 30-50 nt, etc.

In some embodiments, the miRNA-2911 and the nucleic acid molecule derived therefrom are not the following RNA molecule: GGCCGGGGGACGGACUGGGA (SEQ ID NO: 2)

In a second aspect, the present disclosure provides a protected nucleic acid of interest, characterized in that the miRNA-2911 and the nucleic acid molecule derived therefrom of the first aspect or a nucleic acid molecule having the sequence GGCCGGGGGACGGACUGGGA (SEQ ID NO: 2) are conjugated to the 5' end, the 3' end or both the 5'end and the 3' end of the nucleic acid of interest, or inserted into the nucleic acid of interest; and the stability of the nucleic acid of interest is improved.

In some embodiments, the nucleic acid of interest is ≥ 8 nt in length. In some embodiments, the nucleic acid of interest is 8-5000 nt in length, such as 8-4000 nt, 8-3000 nt, 8-2000 nt, 8-1000 nt, 8-500 nt, 8-200 nt, 8-150 nt, 8-100 nt, 8-80 nt, 8-50 nt, 8-40 nt, 8-30 nt, 8-20 nt, 8-10 nt, 30-180 nt, 20-100 nt, and 30-50 nt, etc.

In some embodiments, the nucleic acid of interest is a nucleic acid for gene knockdown, a nucleic acid for gene knockout, a nucleic acid for gene activation, a nucleic acid for gene modification, a nucleic acid for gene editing, a nucleic acid for gene regulation, a nucleic acid for protein regulation, a nucleic acid for protein expression, a nucleic acid for bioassay or a nucleic acid drug.

In some embodiments, the nucleic acid of interest is an oligonucleotide, a single-stranded DNA, a double-stranded DNA, a single-stranded RNA, a double-stranded RNA, an mRNA, or an ncRNA (non-coding RNA).

In some embodiments, the oligonucleotide is an antisense oligonucleotide (ASO) or an apatmer. In some embodiments, the ncRNA is miRNA (microRNA), siRNA (small interfering RNA), saRNA (small activating RNA), piRNA (Piwi protein-interacting RNA), lncRNA (long non-coding RNA), circRNA (circular RNA), fragments thereof, or other regulatory RNA.

In some embodiments, the nucleic acid of interest is DNA or RNA.

In some embodiments, the nucleic acid of interest is modified or unmodified. In some embodiments, the miRNA-2911 and the nucleic acid molecule derived therefrom are modified or unmodified. In some embodiments, the conjugation is a chemical covalent linkage, preferably a linkage via a phosphodiester bond.

In some embodiments, the method for constructing the protected nucleic acid of interest includes chemical synthesis, genetic engineering based on the principle of PCR or biosynthesis.

In a third aspect, the present disclosure provides a pharmaceutical composition, characterized in that the pharmaceutical composition comprises the protected nucleic acid of interest of the second aspect and a pharmaceutically acceptable carrier.

In a fourth aspect, the present disclosure provides a method for improving the stability of a nucleic acid of interest, characterized in that the miRNA-2911 and the nucleic acid molecule derived therefrom of the first aspect or a nucleic acid having the sequence GGCCGGGGGACGGACUGGGA (SEQ ID NO: 2) are conjugated to the 5' end, the 3' end or both the 5'end and the 3' end of the nucleic acid of interest, or inserted into the nucleic acid of interest.

In some embodiments, the nucleic acid of interest is ≥ 8 nt in length, for example, 8-5000 nt such as 8-4000 nt, 8-3000 nt, 8-2000 nt, 8-1000 nt and 8-500 nt, preferably 8-200 nt such as 8-150 nt, 8-100 nt, 8-80 nt, 8-50 nt, 8-40 nt, 8-30 nt, 8-20 nt, 8-10 nt, 30-180 nt, 20-100 nt, and 30-50 nt, etc.

In some embodiments, the nucleic acid of interest is a nucleic acid for gene knockdown, a nucleic acid for gene knockout, a nucleic acid for gene activation, a nucleic acid for gene modification, a nucleic acid for gene editing, a nucleic acid for gene regulation, a nucleic acid for protein regulation, a nucleic acid for protein expression, a nucleic acid for bioassay or a nucleic acid drug.

In some embodiments, the nucleic acid of interest is an oligonucleotide, a single-stranded DNA, a double-stranded DNA, a single-stranded RNA, a double-stranded RNA, an mRNA or an ncRNA.

In some embodiments, the oligonucleotide is an antisense oligonucleotide ASO or an apatmer.

In some embodiments, the ncRNA is miRNA, siRNA, saRNA, piRNA, lncRNA, circRNA, fragments thereof, or other regulatory RNAs.

In some embodiments, the nucleic acid of interest is modified or unmodified. In some embodiments, the miRNA-2911 and the nucleic acid molecule derived therefrom are modified or unmodified.

In some embodiments, the conjugation is a chemical covalent linkage, preferably a linkage via a phosphodiester bond.

In some embodiments, the method for constructing the protected nucleic acid of interest includes chemical synthesis, genetic engineering based on the principle of PCR or biosynthesis. In some embodiments, the nucleic acid of interest is DNA or RNA.

Embodiments of the present disclosure are set forth in detail in the following description and examples. It should be understood that the present disclosure is not limited to the specific embodiments described herein and thereby may be modified. Numerous modifications or variations are possible and obtained by those skilled in the art, and all such modifications and variations are within the scope of the disclosure.

### BRIEF DESCRIPTION OF THE DRAWINGS

The above is only an overview of the technical solutions of the present disclosure. The present disclosure will be further illustrated in detail below in combination with the accompanying drawings and specific embodiments to make the technical means of the present disclosure to be understood more clearly.
Figure 1 shows the stability of an RNA drug containing miRNA-2911 in serum.
Figure 2 shows the stability of RNA drugs containing miRNA-2911-based modified RNA in serum.
Figure 3 shows the stability of a nucleic acid drug chimera containing miRNA-2911-based modified RNA in serum.
Figure 4 shows the stability of DNA drugs containing miRNA-2911-based modified DNA in serum.
Figure 5 shows the stability of a nucleic acid drug chimera containing miRNA-2911-based modified DNA in serum.
Figure 6 shows the stability of R2911A containing nucleic acid drugs with different sequences in serum.
Figure 7 shows the stability of R2911A containing nucleic acid drugs of different lengths in serum.
Figure 8 shows the stability results of nucleic acid drugs containing R2911A in animals.
Figure 9 shows the stability of nucleic acid drugs containing R2911A at the 5' end or 3' end and inside in serum.
Figure 10 evaluates the ability of a siRNA containing R2911A to knock down the target RNA in cells (Hep3B and 97H cell lines).
Figure 11 evaluates the effect of an mRNA containing R2911A on protein expression in cells.

### DETAILED DESCRIPTION OF THE INVENTION

Several aspects of the present disclosure are described below with reference to exemplary applications for illustration. It should be understood that many specific details, relationships, and methods are set forth to provide a thorough understanding of the present disclosure. However, those skilled in the relevant art will readily recognize that the present disclosure may be practiced without one or more of the specific details or otherwise.

The methods for protecting nucleic acid drugs from degradation in the prior art include chemical and non-chemical modifications, but both have their limitations. The inventors of the present application pioneered the use of miRNA-2911 and a derivative thereof to protect nucleic acid drugs, so as to significantly enhance their stability in serum.

Compared with the prior art, the present disclosure has the following beneficial effects:
1. The nucleic acid molecules and methods of the present disclosure are capable of enhancing the stability of nucleic acid drugs in serum without any modification;
2. It has been verified that the nucleic acid drug of the present disclosure has the advantage of high stability in serum compared to the existing conventional protection methods via chemically and non-chemically modification; and
3. The protection methods of the present disclosure, in addition to being applied to oligonucleotide drugs, can also be used to protect ncRNAs and mRNAs, so as to significantly improve the stability of ncRNAs and mRNAs, which would provide strong support for the development of ncRNA and mRNA drugs.

The terms used herein are for the purpose of describing particular embodiments only and are not intended to limit the present disclosure. As used herein, the singular forms "a", "an" and "the" are intended to include the plural forms as well, unless otherwise defined herein. In addition, the open-ended expressions "include" and "comprise" are interpreted as also containing structural components or steps that are not described. However, it should be noted that the open-ended expressions also cover the case where only the described components and method steps are included (that is, the case of using the closed expression "consist of').

As used throughout, a range is used as a shorthand for describing each and all values within the range. Any value within a range, such as an integer value, may be selected as the end point of the range. For example, the expression of no more than 10 nucleotides means no more than 1, 2, 3, 4, 5, 6, 7, 8, 9, and 10 nucleotides and any subranges formed by them, such as 1-8 nt, 2-5 nt, 3-7 nt, etc.

All scientific and technical terms recited in the description shall have the same meanings as commonly understood by those skilled in the art. The definitions in the description shall prevail in case of conflict. Some terms are explained below to make the description of the present disclosure easier to understand.

In the present disclosure, the terms "nucleic acid", "nucleic acid molecule" and "nucleic acid drug" may be used interchangeably, to refer to any DNA, RNA or DNA/RNA chimera, and may be an oligonucleotide or a polynucleotide, and may be unmodified RNA or DNA, or modified RNA or DNA. These terms include but are not limited to, single-stranded and double-stranded DNA, DNA that is a mixture of single-stranded and double-stranded regions, single-stranded and double-stranded RNA, RNA that is a mixture of single-stranded and double-stranded regions, and hybrid molecules containing DNA and RNA that may be single-stranded or double-stranded or a mixture of single-stranded and double-stranded regions.

In some embodiments, a nucleic acid may comprise one or more modified nucleotides, modified linkages, and the like. Examples of modified linkages or internucleotide linkages include phosphorothioates, phosphorodithioates, and the like. In some embodiments, the nucleotides comprise phosphorus derivatives. Phosphorus derivatives (or modified phosphate groups) that may be attached to sugar or sugar analog moieties in the modified nucleotides of the present disclosure may include monophosphate, diphosphate, triphosphate, alkyl phosphate, alkane phosphate, phosphorothioate, and the like. Methods for preparing of the above mentioned phosphate analogs, as well as incorporating them into nucleotides, modified nucleotides and oligonucleotides are also known and need not be described herein.

As used herein, "unmodified" or "natural" nucleotides include adenine (A), guanine (G), thymine (T), cytosine (C) and uracil (U). Modified nucleotides include nucleotides that are only infrequently or transiently present in natural nucleic acids, for example, hypoxanthine, 6-methyladenine, 5-Me pyrimidine, especially 5-methylcytosine (also known as 5-methyl-2' deoxycytosine, which often referred to in the art as 5-Me-C), 5-hydroxymethylcytosine (HMC), glycosylated HMC, gentiobiosyl HMC, and synthetic nucleotides, for example, 2-aminoadenine, 2-(methylamino)adenine, 2-(imidazolylalkyl)adenine, 2-(aminoalkylamino)adenine, or other heterosubstituted alkyladenine, 2-thiouracil, 2-thiothymine, 5-bromouracil, 5-hydroxymethyluracil, 8-azaguanine, 7-deazaguanine, N6 (6-aminohexyl) adenine and 2,6-diaminopurine. "Generic" bases known in the art, e.g., inosine, may be included.

### Nucleic Acid Modification

### (1) Nucleic acid structure.

In some embodiments, the nucleic acid (e.g., RNA targeting DNA) of the present disclosure comprises one or more modification(s) (e.g., base modification, backbone modification, etc.) to provide new or enhanced properties (e.g., improved stability) for the nucleic acid. As known in the art, nucleosides are base-sugar combinations. The base moiety of a nucleoside is generally a heterocyclic base. Purines and pyrimidines are the two most common types of such heterocyclic bases. A nucleotide can be a nucleoside that further comprises a phosphate group covalently linked to the sugar moiety of the nucleoside. For those nucleosides comprising pentofuranose, the phosphate group can be linked to the 2', 3' or 5' hydroxyl moiety of the sugar. In the formation of oligonucleotides, nucleosides adjacent to each other are covalently linked by the phosphate groups to form linear polymeric compounds. In turn, the respective ends of the linear polymeric compounds can be further linked to form a cyclic compound. However, linear compounds are generally preferred. In addition, linear compounds may have internal nucleotide-base complementarity and thus may be folded in such a way as to produce fully or partially double-stranded compounds. Within oligonucleotides, the phosphate group is commonly referred to as the internucleoside backbone that forms the oligonucleotide. The normal bond or backbone of RNA and DNA is 3' to 5' phosphodiester linkage.

### (2) Modified backbones and internucleoside linkages.

Examples of preferred nucleic acids with modifications include nucleic acids with modified backbones or non-natural internucleoside linkages. Nucleic acids (with modified backbones) include those that retain phosphorus atoms in the backbone and those that do not have a phosphorus atom in the backbone.

Preferred modified oligonucleotides containing a phosphorus atom include, for example, phosphorothioates, chiral phosphorothioates, phosphorodithioates, phosphotriesters, aminoalkylphosphotriesters, methyl and other alkyl phosphates including 3'-alkylene phosphates, 5'-alkylene phosphates, and chiral phosphates, phosphonates, phosphoramidates including 3'-amino phosphoramidate and aminoalkylphosphoramidates, diaminophosphate, thionophosphoramidates, thionoalkylphosphonates, thionoalkylphosphotriesters, borophosphates and selenophosphates having normal 3'-5' linkages, 2'-5'-linked analogs thereof, and those having inverted polarity; wherein one or more internucleotide linkages are 3' to 3', 5' to 5' or 2' to 2' linkages. Preferred nucleic acids having inverted polarity comprise a single 3' to 3' linkage at the 3' internucleotide linkage, that is, it may be a single inverted nucleoside residue without base (the nucleobase is missing or substituted by a hydroxyl group). Various salts (such as potassium or sodium salts), mixed salts and free acid forms are also included.

In some embodiments, the nucleic acid of the present disclosure comprises one or more phosphorothioate linkages and/or heteroatomic internucleoside linkages, specifically -CH₂-NH-O-CH₂-, -CH₂-N(CH₃)-O-CH₂- (known as methylene(methylimino) or MMI backbone), - CH₂-O-N(CH₃)-CH₂-, -CH₂-N(CH₃)-N(CH₃)-CH₂-, and -O-N(CH₃)-CH₂-CH₂- (in which the natural phosphodiester internucleoside linkage is represented as -O-P(=O)(OH)-O-CH₂-).

Other backbone modifications also include, for example, nucleic acids having morpholino backbone structures. For example, in some embodiments, the nucleic acid of the disclosure comprises a six-membered morpholino ring in place of a ribose ring. In some of these embodiments, phosphodiester linkages are replaced by diaminophosphate or other non-phosphodiester internucleoside linkages.

A preferred modified polynucleotide backbones without a phosphorus atom have backbones formed by short-chain alkyl or cycloalkyl internucleoside linkages, mixed heteroatomic and alkyl or cycloalkyl internucleoside linkages, or one or more short-chain heteroatomic or heterocyclic internucleoside linkages. These backbones include those having morpholino linkages (partially formed from the sugar moiety of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulphonate and sulfonamide backbones; amide backbones; and other backbones having mixed N, O, S and CH₂ components.

Another backbone modification includes a locked nucleic acid (LNA), in which 2'-hydroxyl is linked to 4' carbon atom of the sugar ring to form a 2'-C, 4'-C-oxymethylene linkage, thereby forming a bicyclic sugar moiety. The chain can be methylene (-CH₂-) (a group bridging 2' oxygen atom and 4' carbon atom), wherein n is 1 or 2 (Singh et al., Chem.Commun., 1998, 4, 455-456). LNA and LNA analogues show very high duplex thermal stability with complementary DNA and RNA (Tₘ =+3°C to +10°C), stability towards 3'-nucleic acid exonucleolytic degradation and good solubility.

The synthesis and preparation of LNA adenine, cytosine, guanine, 5- methyl-cytosine, thymine and uracil monomers, together with their oligomerization and nucleic acid recognition properties, have been described in the prior art (Koshkin et al., Tetrahedron, 1998, 54, 3607-3630).

### (3) Modified sugar moiety.

The nucleic acid of the present disclosure may further include one or more substituted sugar moieties. Preferred polynucleotides comprise sugar substituents selected from: OH; F; O-, S- or N-alkyl; O-, S- or N-alkenyl; O-, S- or N-alkynyl; or O-alkyl-O-alkyl, wherein the alkyl, alkenyl and alkynyl may be substituted or unsubstituted C1-C10 alkyl or C2-C10 alkenyl and alkynyl. Particularly preferred sugar substituents include O((CH₂)ₙO)ₘCH₃, O(CH₂)ₙOCH₃, O(CH₂)ₙNH₂, O(CH₂)ₙCH₃, O(CH₂)ₙONH₂ and O(CH₂)ₙON((CH₂)ₙCH₃)₂, wherein n and m are from 1 to about 10. Other preferred polynucleotides comprise sugar substituents selected from C1-C10 lower alkyl, substituted lower alkyl, alkenyl, alkynyl, alkylaryl, aralkyl, O-alkylaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkylaryl, aminoalkylamino, polyalkylamino, substituted silyl, RNA cleaving groups, reporter groups, intercalators, groups for improving the pharmacokinetic properties of nucleic acids, or groups for improving the pharmacodynamic properties of nucleic acids, and other substituents having similar properties. Preferred modifications include 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504), *i.e.*, an alkoxy-alkoxy group. Another preferred modification includes 2'-dimethylaminooxyethoxy, *i.e.*, a O(CH₂)₂ON(CH₃)₂ group, also known as 2'-DMAOE, as described in the examples hereinafter, and 2'-dimethylaminoethoxyethoxy (also known as 2'-O-dimethyl-amino-ethoxyl-ethyl or 2'-DMAEOE in the art), *i.e.*, 2'-O-CH₂-O-CH₂-N(CH₃)₂.

Other preferred sugar substituents include methoxyl (-O-CH₃), aminopropyloxyl (-OCH₂CH₂CH₂NH₂), allyl (-CH₂-CH=CH₂), -O-allyl (-O-CH₂-CH=CH₂) and fluoro (F). The 2'-sugar substituent may be in the arabinose (upper) position or ribose (lower) position. A preferred 2'-arabino modification is 2'-F. Similar modifications may also be made at other positions on the oligomeric compound, specifically at the 3' terminal nucleoside or at the 3' position of sugar and the 5' position of the 5' terminal nucleotide in the 2'-5' linked oligonucleotides. Oligomeric compound may also have sugar mimetics such as cyclobutyl moieties in place of the pentofuranose.

### (4) Base modification and substitution.

The nucleic acid of the present disclosure may also include modification or substitution of nucleobase (often referred to in the art simply as "base"). As used herein, "unmodified" or "natural" nucleobases include purine bases adenine (A) and guanine (G), and pyrimidine bases thymine (T), cytosine (C) and uracil (U). Modified nucleobases include other synthetic and natural nucleobases, such as 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halo uracil and cytosine, 5-propynyl (-C=C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo (particularly 5-bromo), 5-trifluoromethyl and other 5-substituted uracils and cytosines, 7-methyl guanine and 7-methyl adenine, 2-F-adenine, 2-aminoadenine, 8-azaguanine and 8-azaadenine, 7-deazaguanine and 7-deazaadenine, and 3-deazaguanine and 3-deazaadenine. Other modified nucleobases include tricyclic pyrimidine such as phenoxazine cytidine (1H-pyrimido(5,4-b)(1,4)benzoxazine-2(3H)-one) and phenothiazine cytidine (1H-pyrimido(5,4-b)(1,4)benzothiazine-2(3H)-one), G-clips such as substituted phenazine cytidine (e.g. 9-(2-aminoethoxy)-H-pyrimido(5,4-(b)(1,4)benzoxazine-2(3H)-one), carbazole cytidine (2H-pyrimido(4,5-b)indole-2-one), pyridoindole cytidine (H-pyrido(3',2':4,5)pyrrolo(2,3-d)pyrimidine-2-one).

Heterocyclic base moieties may also include those in which purine or pyrimidine bases are replaced by other heterocycles, such as 7-deazaadenine, 7-deazaguanosine, 2-aminopyridine and 2-pyridone. Other nucleobases include those disclosed in The Concise Encyclopedia of Polymer Science and Engineering, pp. 858-859, Kroschwitz, J. L, ed. John Wiley & Sons, 1990, those disclosed by Angewandte Chemie, International edition, 1991, 30, 613, and those disclosed by Sanghvi,Y.S, Chapter 15, Antisense Research and Applications, pages 289-302, Crooke, S. T. and Lebleu, B., Ed., CRC Press, 1993. Certain of these nucleobases are useful for increasing the binding affinity of the oligomeric compounds. These nucleobases include 5-substituted pyrimidine, 6-azapyrimidine and N-2, N-6 and O-6 substituted purines, including 2-aminopropyladenine, 5-propynyl uracil and 5-propynyl cytosine. 5-methylcytosine substitutions have been shown to increase nucleic acid duplex stability by 0.6°C-1.2°C (Sanghvi et al., Eds., Antisense Research and Applications, CRC Press, Boca Raton, 1993, pages 276-278) and are preferred base substitutions, for example when combined with 2'-O-methoxyethyl sugar modifications.

### (5) Conjugates.

Another possible modification of the nucleic acid of the present disclosure involves chemically linking one or more moieties or conjugates that enhance the activity, cellular distribution or cellular uptake of oligonucleotides to a polynucleotide. These moieties or conjugates may include conjugate groups that covalently bind functional groups such as primary hydroxyl groups or secondary hydroxyl groups. Conjugate groups include, but are not limited to, intercalators, reporter molecules, polyamines, polyamides, polyethylene glycols, polyethers, groups for enhancing the pharmacodynamic properties of oligomers, and groups for enhancing the pharmacokinetic properties of oligomers. Preferred conjugate groups include, but are not limited to, cholesterols, lipids, phospholipids, biotin, phenazine, folate, phenanthridine, anthraquinone, acridine, fluorescein, rhodamine, coumarin and dyes. The groups for enhancing pharmacodynamic properties include groups for improving uptake, enhancing resistance to degradation, and/or enhancing sequence-specific hybridization with nucleic acids of interest. The groups for enhancing pharmacokinetic properties include groups for improving uptake, distribution, metabolism or excretion of the nucleic acid of the present disclosure.

Conjugate moieties include, but are not limited to: lipid moieties such as a cholesterol moiety (Letsinger et al., Proc. Natl. Acid. Sci. USA, 1989, 86, 6553-6556), cholic acid (Manoharan et al., Bioorg. Med. Chem. Let., 1994, 4, 1053-1060), a thioether, *e.g.*, hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, *e.g.*, dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75, 49-54), a phospholipid, *e.g.*, di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

In some embodiments, the nucleic acid molecules of the disclosure comprise or consist of SEQ ID NO: 1 or 3. In some embodiments, the nucleic acid molecules of the present disclosure comprise or consist of two or more of SEQ ID NO: 1 or SEQ ID NO: 3 directly linked or indirectly linked by a linker (*i.e.*, a dimer, trimer, and the like of SEQ ID NO: 1 or SEQ ID NO: 3).

In the present disclosure, the terms "linker group" and "linker" may be used interchangeably, and refer to an oligonucleotide sequence used to link different parts of a sequence. The oligonucleotide sequence may comprise unmodified or modified natural or non-natural nucleotides. In some embodiments, the linker group or linker comprises A, T, C, G and/or U. In some embodiments, the linker group or linker comprises G. In some embodiments, the linker group or linker consists of G(s). In some embodiments, the linker group or linker is 1-10 nucleotides in length, such as 1-5 nucleotides, such as 3-5 nucleotides. In some embodiments, the linker group or linker is GGGGG (SEQ ID NO: 30). In some embodiments, the protective effect of the nucleic acid molecules of the disclosure is not affected by the presence of the linker group or linker.

In the present disclosure, the terms "conjugate" and "link" are used interchangeably, and refer to linking by a chemical bond.

As used herein, the stability of the nucleic acid in serum refers to the stability of the nucleic acid in serum of various concentrations such as 50% serum. The improvement of stability herein refers to that the stability of the nucleic acid of interest, for example the stability in serum, is improved compared with the nucleic acid of interest not protected by the nucleic acid molecule of the present disclosure or protected by other methods. In some embodiments, the nucleic acid of interest has equivalent or similar activity to the nucleic acid of interest not protected by the nucleic acid molecule of the present disclosure or protected by other methods, while has improved stability. In some embodiments, the activity of the nucleic acid of interest remains unchanged.

As used herein, the term "pharmaceutically acceptable carrier" is intended to include any and all solvents, dispersion media, coatings, anti-bacterial and anti-fungal agents, isotonic agents and absorption delaying agents, etc. that are compatible with drug delivery. Preferred carriers are described in the latest edition of *Remington's Pharmaceutical Sciences*, a standard reference publication in the art, which is incorporated herein by reference. Preferred examples of such carriers or diluents include, but are not limited to, water, saline, Ringer's solution, dextrose solution, and 5% human serum albumin. The use of such carriers and agents is well known in the art. Unless incompatible with the substances provided herein, any conventional carriers or agents are considered to be used in the composition.

The following specific examples are merely used to illustrate the solution of the present disclosure, but are not intended to limit the technical solution of the present disclosure. Those skilled in the art should understand that the disclosure may still be modified or equivalently replaced. Any modification or partial replacement without departing from the spirit and scope of the present disclosure shall fall within the protection scope of the present disclosure.

### Examples

Unless otherwise specified, the materials used in the examples herein are all commercially available. Various specific experimental methods used herein are conventional experimental methods in the art or in accordance with the steps and conditions suggested by the manufacturer, and can be routinely determined by those skilled in the art as required. In addition, the serum used in the examples of the description is Gibco Premium fetal bovine serum. The degradation system comprises: oligonucleotide drug, 1×PBS, 50% serum and water. The electrophoresis conditions used are 180V, 20 min; the loading amount is 2 µg-5 µg. The miRNA-2911 sequence and its derivatives in the examples of the description are added to both ends of the sequences to be protected, unless otherwise specified.

### Example 1: Improved stability of RNA drugs containing miRNA-2911 in serum.

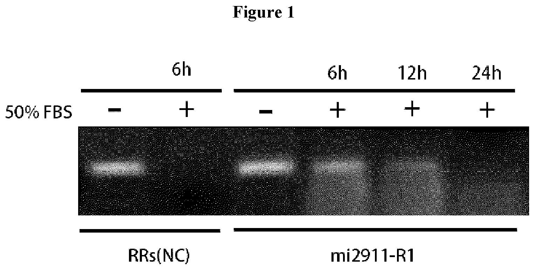

Specific sequence (miRNA-2911) was added to the 5' and 3' end of the RNA (R1) to be protected to form miRNA2911-R1 (shown as mi2911-R1 in Figure 1), in which a random sequence RRs was selected as a negative control (The oligonucleotide is short in length and easily degraded, and thus a random control sequence with basically identical length as the protected sequence was selected, so that their electrophoresis results are almost in a straight line and convenient for comparison). The above sequences were incubated in serum for 6h, 12h and 24h and then analyzed by 3% agarose gel electrophoresis. The results were shown in Figure 1, wherein the random sequence RRs without adding the specific sequence of the present disclosure (miRNA-2911) was used as the negative control (NC), and it was completely degraded at 6h. However, miRNA-2911-R1 still had intact sequences that had not been degraded at 12 h, indicating that miRNA-2911 at both ends greatly improves the ability of R1 against the degradation of nucleases in serum and improves the stability of R1 in serum.

The sequence information of the nucleic acid fragments used in Example 1 is as follows:
miRNA-2911: GGCCGGGGGACGGACUGGGA (SEQ ID NO: 2)
R1_{:} UGAAUGUAGAGAUGCGGUGG (SEQ ID NO: 11)
RRs:

ACCCGACCUCUUCUAUCUGGACCCGACCGUCUCUUUUUUGAGCCCACACUCUACUCG
AC (SEQ ID NO: 12)。

### Example 2: Improving stability of RNA drugs in serum based on miRNA-2911 modified RNAs.

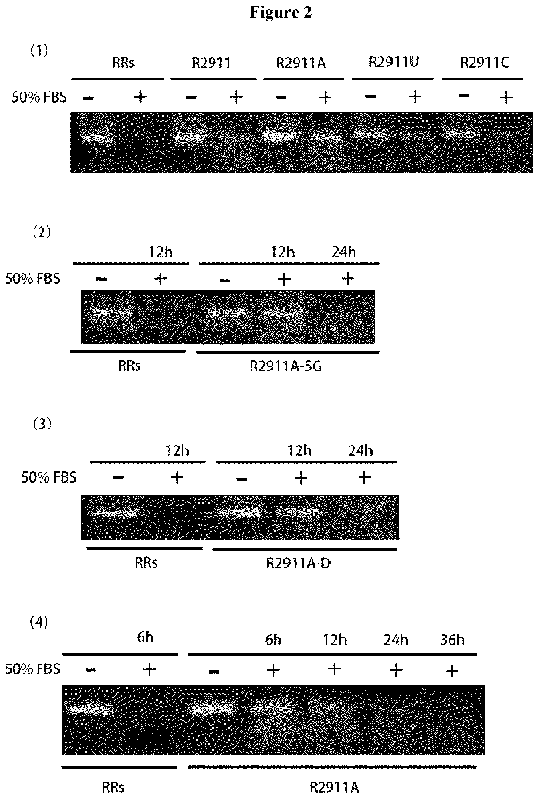

The inventors carried out various modifications to the miRNA-2911 sequence, and then added the modified sequences to both ends of R1 (SEQ ID NO: 11) respectively to evaluate the RNA protection ability of the modified sequences, among which random sequence RRs was selected as a negative control. The above sequences were incubated in serum for 6h, 12h and 24h and then analyzed by 3% agarose gel electrophoresis. The results are shown in Figure 2-(1), Figure 2-(2) and Figure 2-(3), in which the degrees of degradation of different modified nucleic acid drugs in serum are different, but all of them show intact RNAs that have not been degraded, indicating the protective capabilities of the different modified sequences. In addition, the RNA drugs containing miRNA-2911-based modified RNA was incubated in serum for up to 36h, and the results were shown in Figure 2-(4). At 36h, the RNA drugs containing miRNA-2911-based modified RNA still showed intact sequences that had not been degraded, indicating that the RNA drugs containing miRNA-2911-based modified RNA has higher stability in serum.

The sequence information of the nucleic acid fragment used in Example 2 is as follows:
R2911A: GGAAGGGGGAAGGAAAGGGA (SEQ ID NO: 4)
R2911C: GGCCGGGGGCCGGCCCGGGC (SEQ ID NO: 5)
R2911U: GGUUGGGGGUUGGUUUGGGU (SEQ ID NO: 6)
R2911A-5G: GGAAGGGGGGGGGGAAGGAAAGGGA (SEQ ID NO. 7)
R2911A-D: GGAAGGGGGAAGGAAAGGGAGGAAGGGGGAAGGAAAGGGA (SEQ ID NO : 8)
RRs:

ACCCGACCUCUUCUAUCUGGACCCGACCGUCUCUUUUUUGAGCCCACACUCUACUCG
AC (SEQ ID NO: 12).

### Example 3: Modifying miRNA-2911 into DNA to improve the stability of nucleic acid drug chimeras in serum.

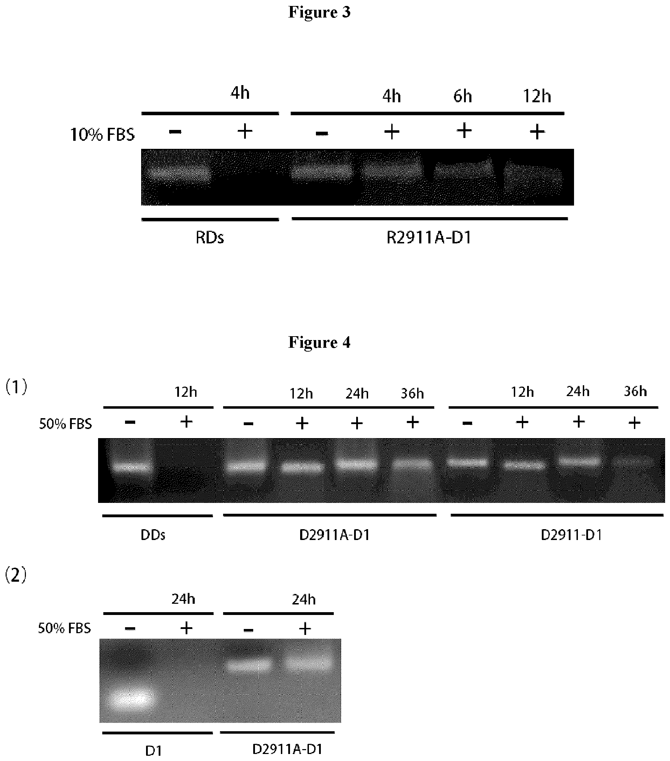

The ability of the modified sequence for protecting DNA was tested by adding the specific sequence R2911A to the 5' and 3' ends of the DNA to be protected (D1) to form R2911A-D1, and random sequence RDs was selected as a negative control. The above sequences were incubated in serum 4h, 6h and 12h and then analyzed by 3% agarose gel electrophoresis. The results were shown in Figure 3, wherein R2911A-D1 still had intact sequences that had not been degraded at 12h, indicating that the nucleic acid drug chimera containing R2911A has higher stability in serum.

The sequence information of D1 and DRs used in Example 3 is as follows:
D1: TGAATGTAGAGATGCGGTGG (SEQ ID NO: 13)
RDs :

UGAAUGUAGAGAUGCGGUGGTGAATGTAGAGATGCGGTGGUGAAUGUAGAGAUGCGG
UGG (SEQ ID NO: 14)。

### Example 4: Modifying miRNA-2911 into DNA to improve the stability of DNA drugs in serum.

The miRNA-2911 sequence was modified into DNA (D2911 and D2911A) and then added to both ends of D1 to form D2911-D1 and D2911A-D1, respectively, for evaluating the DNA protection ability of the modified sequences. Meanwhile, D1 and DDs were used as negative controls. The above sequences were incubated in serum for 12h, 24h and 36h and then analyzed by 3% agarose gel electrophoresis. The results were shown in Figure 4-(1) and 4-(2), wherein the stability of D2911-D1 and D2911A-D1 in serum was significantly enhanced. Figure 4-(2) shows that the enhanced stability of nucleic acid drugs is due to D2911A rather than the sequence itself.

The sequence information of the nucleic acid fragments used in Example 4 is as follows:
D1: TGAATGTAGAGATGCGGTGG (SEQ ID NO: 13)
D2911: GGCCGGGGGACGGACTGGGA (SEQ ID NO: 9)
D2911A: GGAAGGGGGAAGGAAAGGGA (SEQ ID NO: 10)
DDs :

TGAATGTAGAGATGCGGTGGTGAATGTAGAGATGCGGTGGTGAATGTAGAGATGCGGTG
G (SEQ ID NO: 14)。

### Example 5: Modifying miRNA-2911 into DNA to improve the stability of nucleic acid drugs in serum.

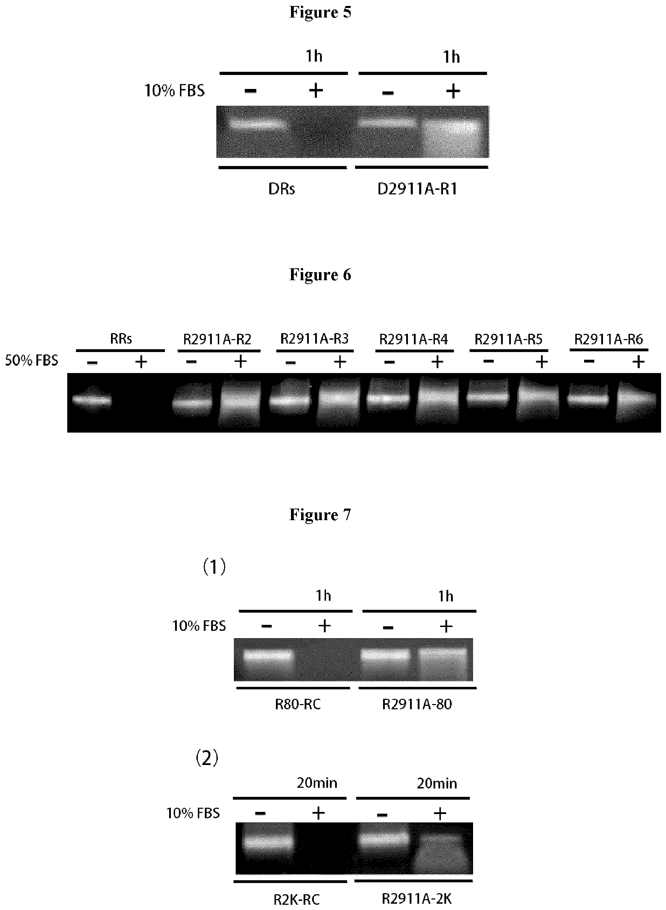

The ability of D2911A for protecting RNA was tested by adding D2911A sequence to both ends of R1 to form D2911A-R1, where random sequence DRs was selected as a negative control. The above sequences were incubated in serum for 1 h and then analyzed by 3% agarose gel electrophoresis. The results were shown in Figure 5, wherein the nucleic acid drug chimera containing D2911A has certain stability in serum.

The sequence information of the nucleic acid fragments used in Example 5 is as follows:
D2911A-R1 : 
GGAAGGGGGAAGGAAAGGGAUGAAUGUAGAGAUGCGGUGGGGAAGGGGGAAGGAA
AGGGA (SEQ ID NO: 15)
DRs:

ATTTGACCTCTTCTATCTGGACCCGACCGUCUCUUUUUUGAGCCCACACTCTACTCGAC
G (SEQ ID NO: 16)。

### Example 6: Evaluating the stability of nucleic acid drugs of different sequences containing R2911A in serum.

The ability of R2911A for protecting nucleic acid drugs of different sequences was tested. Specifically, nucleic acids R2-R6 of different sequences were selected. R2911A was added to both ends of R2-R6 to form R2911A-R2, R2911A-R3, R2911A-R4, R2911A-R5, and R2911A-R6, respectively, wherein random sequence RRs served as a negative control, which was not protected by using the method of the disclosure. The above sequences were incubated in serum for 6h and then analyzed by 3% agarose gel electrophoresis. The results were shown in Figure 6, in which the degrees of degradation of nucleic acid drugs of different sequences containing R2911A in serum were different, but all of them showed intact DNAs that had not been degraded. Combined with the protective ability to R1 in Example 1, the results showed that the nucleic acid drugs of different sequences containing R2911A each were stable in serum, indicating that the method of the present disclosure is universally applicable to the protection of nucleic acid sequences.

The sequence information of the nucleic acid fragments used in Example 6 is as follows:
R2: ACGGGGUCAUUAGUUCAUAG (SEQ ID NO: 17)
R3: UAAGAUACACCUGCAAAGGC (SEQ ID NO: 18)
R4: GCUCUCCUCAAGCGUAUUCA (SEQ ID NO: 19)
R5: GUCGAGCUGGACGGCGACGU (SEQ ID NO: 20)
R6: UGACCCUGAAGUUCAUCUGC (SEQ ID NO: 21)
RRs:

ACCCGACCUCUUCUAUCUGGACCCGACCGUCUCUUUUUUGAGCCCACACUCUACUCG
AC (SEQ ID NO: 12)。

### Example 7: Evaluating the stability of nucleic acid drugs of different lengths containing R2911A in serum.

The ability of R2911A for protecting nucleic acid drugs of different lengths was tested. Specifically, nucleic acids of different lengths were selected, represented by RNAs of 80 nt (R80) and 2000 nt (R2K) in length. R2911A was added to both ends of nucleic acid drugs to form R2911A-R80 and R2911A-R1K, respectively, wherein RNAs without any protection were used as their negative controls (R80-RC and R2K-RC, respectively). The RNA of 2000nt was used to validate the ability of the method of the present disclosure for protecting long-chain RNA (including mRNA and lncRNA). The above sequences were incubated in 10% serum for 1 h and 20 min, respectively, and then analyzed by 3% agarose gel electrophoresis. The results were shown in Figure 7-(1) and Figure 7-(2), in which the degrees of degradation of nucleic acids of different lengths containing R2911A in serum were different, but all of them showed intact RNAs that had not been degraded. Combined with the ability for protecting 20 nt nucleic acids in Example 6, it showed that nucleic acid drugs of different lengths containing RNA R2911A each were stable. In addition, the ability of R2911A for protecting RNA with a length of 5000 nt was also tested. The results showed that R2911A had protective ability to RNA of this length (results not shown).

The sequence information of the nucleic acid fragment used in Example 7 is as follows:
R2911A-R80: 
GGAAGGGGGAAGGAAAGGGAGCUGUGUGACUCCUGCAAAGUGUGGACAACUUCCCA
CGGAGGAAUUCCCGUAUCUAAAGGUGCAGCUGUAUCUUGUCUCCGGAAGGGGGAAG
GAAAGGGA (SEQ ID NO: 22)
R80-RC: 
AACCUCUUAGACAGGUGGGAGAUUAUGAUCAGAGUAAAAGGUAAUUACACAUU
UUAUUUCCAGAAAGUCAGGGGUCUAUA (SEQ ID NO: 23)
R2911A-R2K: 
GGAAGGGGGAAGGAAAGGGAUUAUUUUUAAGAGCUGUGGAGUUCUUAAAUAUC
AACCAUGGCACUUUCUCCUGACCCCUUCCCUAGGGGAUUUCAGGAUUGAGAAAUUU
UUCCAUCGAGCCUUUUUAAAAUUGUAGGACUUGUUCCUGUGGGCUUCAGUGAUGGG
AUAGUACACUUCACUCAGAGGCAUUUGCAUCUUUAAAUAAUUUCUUAAAAGCCUCU
AAAGUGAUCAGUGCCUUGAUGCCAACUAAGGAAAUUUGUUUAGCAUUGAAUCUCUG
AAGGCUCUAUGAAAGGAAUAGCAUGAUGUGCUGUUAGAAUCAGAUGUUACUGCUAA
AAUUUACAUGUUGUGAUGUAAAUUGUGUAGAAAACCAUUAAAUCAUUCAAAAUAA
UAAACUAUUUUUAUUAGAGAAUGUAUACUUUUAGAAAGCUGUCUCCUUAUUUAAAU
AAAAUAGUGUUUGUCUGUAGUUCAGUGUUGGGGCAAUCUUGGGGGGGAUUCUUCUC
UAAUCUUUCAGAAACUUUGUCUGCGAACACUCUUUAAUGGACCAGAUCAGGAUUUG
AGCGGAAGAACGAAUGUAACUUUAAGGCAGGAAAGACAAAUUUUAUUCUUCAUAAA
GUGAUGAGCAUAUAAUAAUUCCAGGCACAUGGCAAUAGAGGCCCUCUAAAUAAGGA
AUAAAUAACCUCUUAGACAGGUGGGAGAUUAUGAUCAGAGUAAAAGGUAAUUACAC
AUUUUAUUUCCAGAAAGUCAGGGGUCUAUAAAUUGACAGUGAUUAGAGUAAUACUU
UUUCACAUUUCCAAAGUUUGCAUGUUAACUUUAAAUGCUUACAAUCUUAGAGUGGU
AGGCAAUGUUUUACACUAUUGACCUUAUAUAGGGAAGGGAGGGGGUGCCUGUGGGG
UUUUAAAGAAUUUUCCUUUGCAGAGGCAUUUCAUCCUUCAUGAAGCCAUUCAGGAU
UUUGAAUUGCAUAUGAGUGCUUGGCUCUUCCUUCUGUUCUAGUGAGUGUAUGAGAC
CUUGCAGUGAGUUUAUCAGCAUACUCAAAAUUUUUUUCCUGGAAUUUGGAGGGAUG
GGAGGAGGGGGUGGGGCUUACUUGUUGUAGCUUUUUUUUUUUUUACAGACUUCACA
GAGAAUGCAGUUGUCUUGACUUCAGGUCUGUCUGUUCUGUUGGCAAGUAAAUGCAG
UACUGUUCUGAUCCCGCUGCUAUUAGAAUGCAUUGUGAAACGACUGGAGUAUGAUU
AAAAGUUGUGUUCCCCAAUGCUUGGAGUAGUGAUUGUUGAAGGAAAAAAUCCAGCU
GAGUGAUAAAGGCUGAGUGUUGAGGAAAUUUCUGCAGUUUUAAGCAGUCGUAUUU
GUGAUUGAAGCUGAGUACAUUUUGCUGGUGUAUUUUUAGGUAAAAUGCUUUUUGU
UCAUUUCUGGUGGUGGGAGGGGACUGAAGCCUUUAGUCUUUUCCAGAUGCAACCUU
AAAAUCAGUGACAAGAAACAUUCCAAACAAGCAACAGUCUUCAAGAAAUUAAACUG
GCAAGUGGAAAUGUUUAAACAGUUCAGUGAUCUUUAGUGCAUUGUUUAUGUGUGG
GUUUCUCUCUCCCCUCCCUUGGUCUUAAUUCUUACAUGCAGGAACACUCAGCAGACA
CACGUAUGCGAAGGGCCAGAGAAGCCAGACCCAGUAAGAAAAAAUAGCCUAUUUAC
UUUAAAUAAACCAAACAUUCCAUUUUAAAUGUGGGGAUUGGGAACCACUAGUUCUU
UCAGAUGGUAUUCUUCAGACUAUAGAAGGAGCUUCCAGUUGAAUUCACCAGUGGAC
AAAAUGAGGAAAACAGGUGAACAAGCUUUUUCUGUAUUUACAUACAAAGUCAGAUC
 
AGUUAUGGGACAAUAGUAUUGAAUAGAUUUCAGCUUUAUGCUGGAGUAACUGGCAU
GUGAGCAAACUGUGUUGGCGUGGGGGUGGAGGGGUGAGGUGGGCGCUAAGCCUUUU
UUUAAGAUUUUUCAGGUACCCCUCACUAAAGGCACCGAAGGCUUAAAGUAGGACAA
CCAUGGAGCCGGAAGGGGGAAGGAAAGGGA (SEQ ID NO: 24)
R2K-RC:

UUAUUUUUAAGAGCUGUGGAGUUCUUAAAUAUCAACCAUGGCACUUUCUCCUG
ACCCCUUCCCUAGGGGAUUUCAGGAUUGAGAAUUUUCCAUCGAGCCUUUUUAAA
AUUGUAGGACUUGUUCCUGUGGGCUUCAGUGAUGGGAUAGUACACUUCACUCAGAG
GCAUUUGCAUCUUUAAAUAAUUUCUUAAAAGCCUCUAAAGUGAUCAGUGCCUUGAU
GCCAACUAAGGAAAUUUGUUUAGCAUUGAAUCUCUGAAGGCUCUAUGAAAGGAAUA
GCAUGAUGUGCUGUUAGAAUCAGAUGUUACUGCUAAAAUUUACAUGUUGUGAUGUA
AAUUGUGUAGAAAACCAUUAAAUCAUUCAAAAUAAUAAACUAUUUUUAUUAGAGA
AUGUAUACUUUUAGAAAGCUGUCUCCUUAUUUAAAUAAAAUAGUGUUUGUCUGUAG
UUCAGUGUUGGGGCAAUCUUGGGGGGGAUUCUUCUCUAAUCUUUCAGAAACUUUGU
CUGCGAACACUCUUUAAUGGACCAGAUCAGGAUUUGAGCGGAAGAACGAAUGUAAC
UUUAAGGCAGGAAAGACAAAUUUUAUUCUUCAUAAAGUGAUGAGCAUAUAAUAAU
UCCAGGCACAUGGCAAUAGAGGCCCUCUAAAUAAGGAAUAAAUAACCUCUUAGACA
GGUGGGAGAUUAUGAUCAGAGUAAAAGGUAAUUACACAUUUUAUUUCCAGAAAGUC
AGGGGUCUAUAAAUUGACAGUGAUUAGAGUAAUACUUUUUCACAUUUCCAAAGUUU
GCAUGUUAACUUUAAAUGCUUACAAUCUUAGAGUGGUAGGCAAUGUUUUACACUAU
UGACCUUAUAUAGGGAAGGGAGGGGGUGCCUGUGGGGUUUUAAAGAAUUUUCCUUU
GCAGAGGCAUUUCAUCCUUCAUGAAGCCAUUCAGGAUUUUGAAUUGCAUAUGAGUG
CUUGGCUCUUCCUUCUGUUCUAGUGAGUGUAUGAGACCUUGCAGUGAGUUUAUCAG
CAUACUCAAAAUUUUUUUCCUGGAAUUUGGAGGGAUGGGAGGAGGGGGUGGGGCUU
ACUUGUUGUAGCUUUUUUUUUUUUUACAGACUUCACAGAGAAUGCAGUUGUCUUGA
CUUCAGGUCUGUCUGUUCUGUUGGCAAGUAAAUGCAGUACUGUUCUGAUCCCGCUG
CUAUUAGAAUGCAUUGUGAAACGACUGGAGUAUGAUUAAAAGUUGUGUUCCCCAAU
GCUUGGAGUAGUGAUUGUUGAAGGAAAAAAUCCAGCUGAGUGAUAAAGGCUGAGU
GUUGAGGAAAUUUCUGCAGUUUUAAGCAGUCGUAUUUGUGAUUGAAGCUGAGUACA
UUUUGCUGGUGUAUUUUUAGGUAAAAUGCUUUUUGUUCAUUUCUGGUGGUGGGAG
GGGACUGAAGCCUUUAGUCUUUUCCAGAUGCAACCUUAAAAUCAGUGACAAGAAAC
AUUCCAAACAAGCAACAGUCUUCAAGAAAUUAAACUGGCAAGUGGAAAUGUUUAAA
CAGUUCAGUGAUCUUUAGUGCAUUGUUUAUGUGUGGGUUUCUCUCUCCCCUCCCUU
GGUCUUAAUUCUUACAUGCAGGAACACUCAGCAGACACACGUAUGCGAAGGGCCAG
AGAAGCCAGACCCAGUAAGAAAAAAUAGCCUAUUUACUUUAAAUAAACCAAACAUU
CCAUUUUAAAUGUGGGGAUUGGGAACCACUAGUUCUUUCAGAUGGUAUUCUUCAGA
CUAUAGAAGGAGCUUCCAGUUGAAUUCACCAGUGGACAAAAUGAGGAAAACAGGUG
AACAAGCUUUUUCUGUAUUUACAUACAAAGUCAGAUCAGUUAUGGGACAAUAGUAU
UGAAUAGAUUUCAGCUUUAUGCUGGAGUAACUGGCAUGUGAGCAAACUGUGUUGGC
GUGGGGGUGGAGGGGUGAGGUGGGCGCUAAGCCUUUUUUUAAGAUUUUUCAGGUAC
CCCUCACUAAAGGCACCGAAGGCUUAAAGUAGGACAACCAUGGAGCC (SEQ ID NO:
25).

### Example 8: Evaluating the stability of nucleic acid drugs containing R2911A in animals.

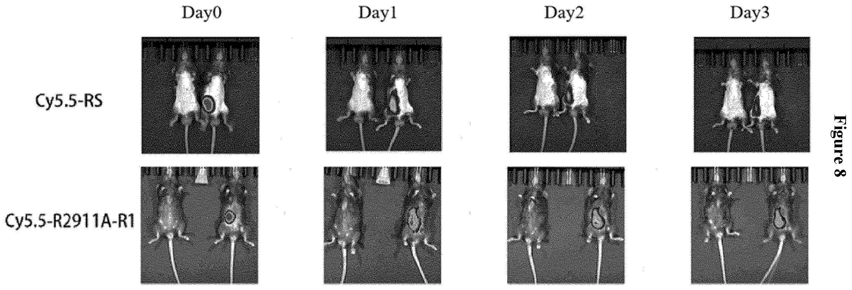

The nucleic acid drugs containing R2911A (R2911A-R1, formed by adding R2911A sequences to both ends of R1) was labeled by cy5.5 to form Cy5.5-R2911A-R1. The Cy5.5-R2911A-R1 was diluted with normal saline before injection. The mice were weighed, treated with abdominal depilation and administered at an injection dose of 2 mg/kg. The mice were injected subcutaneously as follow: grabbing and fixing the mice, injecting at the skin between the two hind limbs, drawing a volume of drug according to the body weight with a syringe, then gently piercing the skin with the bevel of the needle facing upward, and injecting slowly after subcutaneously inserting the needle about 1-2 cm along the skin. After the successful subcutaneous injection, the skin of mice would bulge at the injection site, and the injection was completed by smoothly withdrawing the needle in the opposite direction of entry. *In vivo* imaging was performed on 0d, 1d, 2d and 3d after injection to observe the distribution and degradation of Cy5.5-R2911A-R1 in mice after subcutaneous injection. The stability of R2911A-R1 nucleic acid drug in animals was preliminarily investigated. The results were shown in Figure 8. Obvious fluorescence could still be detected in mice injected with R2911A-R1 nucleic acid drug on 3d, while almost no fluorescence could be detected in mice injected with Cy5.5-RRs (labeled with Cy5.5 and without R2911A, shown as Cy5.5-RS in Figure 8) after 3d. This example showed that the nucleic acid drugs containing R2911A had good stability in animals.

### Example 9: Evaluating the stability of nucleic acid drugs containing R2911A at the 5' end, 3' end or internally in serum.

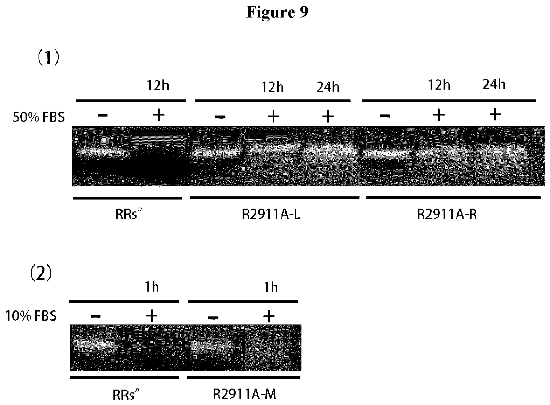

In order to evaluate the stability of nucleic acid drugs containing R2911A at the 5' end, 3' end or internally in serum, R2911A was added at the 5' or 3' end of R1 or inside R1, while RNA without any protection was used as a negative control (RRs"). These sequences were incubated in serum for 12h, 24h and 1h, respectively and then analyzed by 3% agarose gel electrophoresis. The results were shown in Figures 9-(1) and 9-(2). The protective effect was unsatisfactory when R2911A was added internally (Figure 9-(2)). There remained intact sequences that had not been degraded at 24 h when R2911A was added at the 5' or 3' end (Figure 9-(1)), indicating that the R2911A at the 5' or the 3' end could improve the stability of RNA in serum. Combined with the protective ability to R1 in Example 2, it showed that the stability of nucleic acid drugs in serum was increased more significantly when the protective nucleic acid molecule of the present disclosure was present at both ends of R1.

The sequence information of the nucleic acid fragment used in Example 9 is as follows:
R2911A-M: 
UGAAUGUAGAGAUGCGGUGGGAAGGGGAAGGAAAGGAUGAAUGUAGAGAUGC
GGUGG (SEQ ID NO: 26)
R2911A-L: GGAAGGGGGAAGGAAAGGGAUGAAUGUAGAGAUGCGGUGG (SEQ ID NO: 27)
R2911A-R: UGAAUGUAGAGAUGCGGUGGGGAAGGGGGAAGGAAAGGGA (SEQ **ID** NO: 28)
RRs": ACCCGACCUCUUCUAUCUGGACCCGACCGUCUCUUUUUUG (SEQ ID NO: 29)_{°}

### Example 10: Evaluating the knockdown effect of siRNA drugs containing R2911A on target RNA in cells.

1. FDA-approved inclisiran was used as a positive control (the positive control is a chemically modified siRNA), and an unmodified siRNA drug containing R2911A (inclisiran-R2911A) was formed by adding R2911A at the 3' end of its RNA sequence. Lipofectamine 2000 was used for siRNA transfection as follow: an appropriate amount of inclisiran-2911A was diluted with 50 µL of Opti-MEM, while 4.8 µL Lipofectamine 2000 was diluted with 50 µL of Opti-MEM, gently blown to mix, and left for 5 min at room temperature. The obtained Lipofectamine 2000 dilution was then added to the inclisiran-2911A dilution to form a mixture and incubated at room temperature for 20 min. At the same time, the medium of the cells (97H cells and Hep3B cells with 50-60% cell density) in 12-well plates were replaced with antibiotic (penicillin-streptomycin)-free medium (DMEM), added with the mixture dropwise, gently mixed, and replaced with complete culture medium containing serum and antibiotics after 5h to 6h. After the transfection time reaching 48h, 72h, 96h, the cells were collected for extracting total RNA. After 96h of transfection, the cell density was too high to continue culture in 12-well plates, it was necessary to pass the cells into 6-well plates, and collect cells after 120h of transfection time for extracting total RNA.

### 2. Extraction of total cellular RNA:

(1) Discarding the medium, and then rinsing the cells once with pre-cooled 1×PBS.
(2) Adding 0.5 mL of Trizol to the cells in the 12-well plate, adding 1 mL of Trizol to the cells in the 6-well plate, and mixing by blowing with a pipette to completely lyse the cells.
(3) Adding of 20% the total volume of chloroform to the completely lysed Trizol, capping the tube, shaking vigorously to make it fully mixed, letting it stand at room temperature for 2 min, and then centrifuging at 12000×g for 15 min at 4°C.
(4) Pipetting about 200 µL (12-well plate) and 400 µL (6-well plate) of the aqueous phase layer into new EP tubes, adding an equal volume of isopropanol to mix, placing in a -80°C low-temperature refrigerator for half an hour, and then centrifuging at 12000×g for 15 minutes at 4°C.
(5) After the centrifugation, a white precipitate (i.e. RNA) can be seen at the bottom of the tube. Discarding the supernatant, adding 1 mL of 75% ethanol to resuspend the precipitate, and then centrifuging at 7500×g for 10 min at 4°C.
(6) Aspirating off the ethanol carefully without pipetting the precipitate. Leaving the tube at room temperature. After the ethanol being evaporated, adding 50 µL of RNase-free H₂O and incubating at 42°C for 2 min to fully dissolve the RNA. Then taking out a small amount of RNA to measure its concentration, and using TBE electrophoresis to detect the integrity of RNA.
(7) Taking out 1 µg of total RNA for reverse transcription with a reverse transcription system of 20 µL, and obtaining the cDNA.

### 3. Real-time fluorescence quantitative PCR:

(1) Taking out the cDNA from a -20°C refrigerator, dissolving it at room temperature, and then diluting it 10-fold with 180 µL ddH₂O.
(2) The qPCR reaction system was as follows:

| | |
|---|---|
| 2 x qPCR SYRB Green Mix | 20 µl; |
| forward primer (10uM) | 0.5 µl; |
| reverse primer (10uM) | 0.5 µl; |
| cDNA template | 4 µl; |
| ddH₂O | 5 µl. |

The PCR conditions were as follows: 95°C for 3min; 95°C for 10s, 60°C for 10s, 72°C for 20s (this program is 40 cycles); 95°C for 15s; 60°C for 60s; the dissociation curve is 60°C~95°C, and the fluorescence signal is absorbed every 0.5°C rise; 95°C for 10s.

(3) PCR was performed by using ABI step one plus quantitative instrument, and the experimental data were analyzed according to the results by applying the △△Ct algorithm.

The knockdown ability of the siRNA targeting *PCSK9* (inclisiran or inclisiran linked with 2911) used in this example was tested at the cellular level by using 97H and Hep3B cell lines, while an equal volume of PBS group was transfected and set as a negative control (Mock) and inclisiran was set as a positive control.

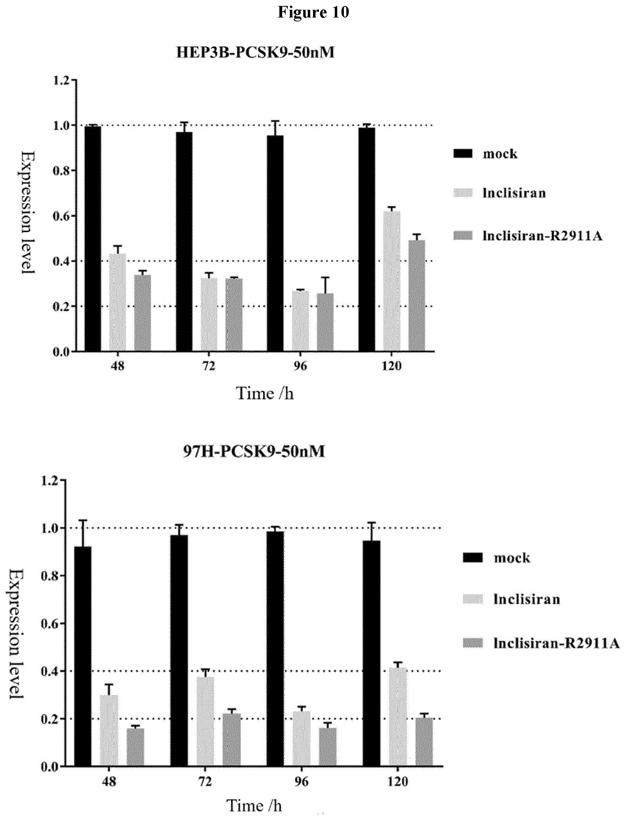

The results **(****Figure 10****)** showed that **the siRNA drug containing R2911A (inclisiran-R2911A) had a good ability to knock down target genes in cells.**

The nucleic acid fragment information used in the example is as follows:
Inclisiran (fully modified sequence): A**C**A*AAA*G*C*A*A*AAC*A*G*G*U*C*UAG*A*A

Underlined nucleotides were subjected to a methoxy modification at the 2' position; nucleotides marked in italics were subjected to a fluoro modification (fluoro is the replacement of the hydroxyl group at the 2' position with a fluorine atom); * indicated thio-modification (indicating the replacement of a non-bridging oxygen atom in phosphate linkage with a sulfur atom).
Inclisiran-R2911A: ACAAAAGCAAAACAGGUCUAGAAAAAGGAAGGGGGAAGGAAAGGGA

### Example 10: Evaluating of the intracellular protein expression of R2911A-containing mRNA.

To evaluate the intracellular protein expression of R2911A-containing mRNA drugs, R2911A was added to both ends of the firefly luciferase gene (mRNA1) to form R-mRNA1. mRNA1 without any protection was used as a negative control (mRRs).

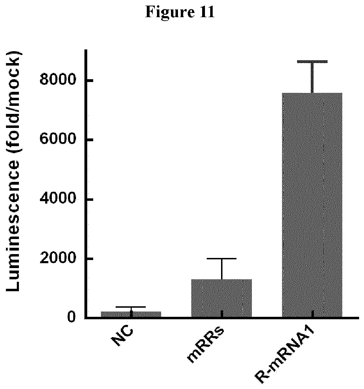

These sequences were transfected into 293U cells after *in vitro* transcription. After 24h, a detection reagent (Promega, ONE-GloUM luciferase detection system, E6110) in equal volume with the culture medium was added and the luminescence signal was detected by microplate reader (Thermo, Scientific VARioskan LUX). The results were shown in **Figure 11****.** When R2911A was added to both ends of mRNA1, the intracellular protein expression level of R-mRNA1 was significantly higher than that of mRRs, indicating that the inclusion of R2911A significantly improved the intracellular protein expression of mRNA drugs.

Wherein, sequence information is as follows:
mRNA1(SEQ ID NO:31): 
UUAAAACAGCCUGUGGGUUGAUCCCACCCACAGGCCCAUUGGGCGCUAG
CACUCUGGUAUCACGGUACCUUUGUGCGCCUGUUUUAUACCCCCUCCCCCAACUG
UAACUUAGAAGUAACACACACCGAUCAACAGUCAGCGUGGCACACCAGCCACGUU
UUGAUCAAGCACUUCUGUUACCCCGGACUGAGUAUCAAUAGACUGCUCACGCGGU
UGAAGGAGAAAGCGUUCGUUAUCCGGCCAACUACUUCGAAAAACCUAGUAACACC
GUGGAAGUUGCAGAGUGUUUCGCUCAGCACUACCCCAGUGUAGAUCAGGUCGAU
GAGUCACCGCAUUCCCCACGGGCGACCGUGGCGGUGGCUGCGUUGGCGGCCUGCC
CAUGGGGAAACCCAUGGGACGCUCUAAUACAGACAUGGUGCGAAGAGUCUAUUG
AGCUAGUUGGUAGUCCUCCGGCCCCUGAAUGCGGCUAAUCCUAACUGCGGAGCAC
ACACCCUCAAGCCAGAGGGCAGUGUGUCGUAACGGGCAACUCUGCAGCGGAACCG
ACUACUUUGGGUGUCCGUGUUUCAUUUUAUUCCUAUACUGGCUGCUUAUGGUGA
CAAUUGAGAGAUCGUUACCAUAUAGCUAUUGGAUUGGCCAUCCGGUGACUAAUA
GAGCUAUUAUAUAUCCCUUUGUUGGGUUUAUACCACUUAGCUUGAAAGAGGUUA
AAACAUUACAAUUCAUUGUUAAGUUGAAUACAGCAAAAUGGCCGAUGCUAAGAA
CAUUAAGAAGGGCCCUGCUCCCUUCUACCCUCUGGAGGAUGGCACCGCUGGCGAG
 
CAGCUGCACAAGGCCAUGAAGAGGUAUGCCCUGGUGCCUGGCACCAUUGCCUUCA
CCGAUGCCCACAUUGAGGUGGACAUCACCUAUGCCGAGUACUUCGAGAUGUCUGU
GCGCCUGGCCGAGGCCAUGAAGAGGUACGGCCUGAACACCAACCACCGCAUCGUG
GUGUGCUCUGAGAACUCUCUGCAGUUCUUCAUGCCAGUGCUGGGCGCCCUGUUCA
UCGGAGUGGCCGUGGCCCCUGCUAACGACAUUUACAACGAGCGCGAGCUGCUGAA
CAGCAUGGGCAUUUCUCAGCCUACCGUGGUGUUCGUGUCUAAGAAGGGCCUGCAG
AAGAUCCUGAACGUGCAGAAGAAGCUGCCUAUCAUCCAGAAGAUCAUCAUCAUG
GACUCUAAGACCGACUACCAGGGCUUCCAGAGCAUGUACACAUUCGUGACAUCUC
AUCUGCCUCCUGGCUUCAACGAGUACGACUUCGUGCCAGAGUCUUUCGACAGGGA
CAAAACCAUUGCCCUGAUCAUGAACAGCUCUGGGUCUACCGGCCUGCCUAAGGGC
GUGGCCCUGCCUCAUCGCACCGCCUGUGUGCGCUUCUCUCACGCCCGCGACCCUA
UUUUCGGCAACCAGAUCAUCCCCGACACCGCUAUUCUGAGCGUGGUGCCAUUCCA
CCACGGCUUCGGCAUGUUCACCACCCUGGGCUACCUGAUUUGCGGCUUUCGGGUG
GUGCUGAUGUACCGCUUCGAGGAGGAGCUGUUCCUGCGCAGCCUGCAAGACUACA
AAAUUCAGUCUGCCCUGCUGGUGCCAACCCUGUUCAGCUUCUUCGCUAAGAGCAC
CCUGAUCGACAAGUACGACCUGUCUAACCUGCACGAGAUUGCCUCUGGCGGCGCC
CCACUGUCUAAGGAGGUGGGCGAAGCCGUGGCCAAGCGCUUUCAUCUGCCAGGCA
UCCGCCAGGGCUACGGCCUGACCGAGACAACCAGCGCCAUUCUGAUUACCCCAGA
GGGCGACGACAAGCCUGGCGCCGUGGGCAAGGUGGUGCCAUUCUUCGAGGCCAAG
GUGGUGGACCUGGACACCGGCAAGACCCUGGGAGUGAACCAGCGCGGCGAGCUGU
GUGUGCGCGGCCCUAUGAUUAUGUCCGGCUACGUGAAUAACCCUGAGGCCACAAA
CGCCCUGAUCGACAAGGACGGCUGGCUGCACUCUGGCGACAUUGCCUACUGGGAC
GAGGACGAGCACUUCUUCAUCGUGGACCGCCUGAAGUCUCUGAUCAAGUACAAGG
GCUACCAGGUGGCCCCAGCCGAGCUGGAGUCUAUCCUGCUGCAGCACCCUAACAU
UUUCGACGCCGGAGUGGCCGGCCUGCCCGACGACGAUGCCGGCGAGCUGCCUGCC
GCCGUCGUCGUGCUGGAACACGGCAAGACCAUGACCGAGAAGGAGAUCGUGGACU
AUGUGGCCAGCCAGGUGACAACCGCCAAGAAGCUGCGCGGCGGAGUGGUGUUCGU
GGACGAGGUGCCCAAGGGCCUGACCGGCAAGCUGGACGCCCGCAAGAUCCGCGAG
AUCCUGAUCAAGGCUAAGAAAGGCGGCAAGAUCGCCGUGUAA
R-mRNA1(SEQ ID NO:32):

GGAAGGGGAAGGAAAGGAUUAAAACAGCCUGUGGGUUGAUCCCACCC
ACAGGCCCAUUGGGCGCUAGCACUCUGGUAUCACGGUACCUUUGUGCGCCUGUUU
UAUACCCCCUCCCCCAACUGUAACUUAGAAGUAACACACACCGAUCAACAGUCAG
CGUGGCACACCAGCCACGUUUUGAUCAAGCACUUCUGUUACCCCGGACUGAGUAU

CAAUAGACUGCUCACGCGGUUGAAGGAGAAAGCGUUCGUUAUCCGGCCAACUACU
UCGAAAAACCUAGUAACACCGUGGAAGUUGCAGAGUGUUUCGCUCAGCACUACCC
CAGUGUAGAUCAGGUCGAUGAGUCACCGCAUUCCCCACGGGCGACCGUGGCGGUG
GCUGCGUUGGCGGCCUGCCCAUGGGGAAACCCAUGGGACGCUCUAAUACAGACAU
GGUGCGAAGAGUCUAUUGAGCUAGUUGGUAGUCCUCCGGCCCCUGAAUGCGGCU
AAUCCUAACUGCGGAGCACACACCCUCAAGCCAGAGGGCAGUGUGUCGUAACGGG
CAACUCUGCAGCGGAACCGACUACUUUGGGUGUCCGUGUUUCAUUUUAUUCCUAU
ACUGGCUGCUUAUGGUGACAAUUGAGAGAUCGUUACCAUAUAGCUAUUGGAUUG
GCCAUCCGGUGACUAAUAGAGCUAUUAUAUAUCCCUUUGUUGGGUUUAUACCAC
UUAGCUUGAAAGAGGUUAAAACAUUACAAUUCAUUGUUAAGUUGAAUACAGCAA
AAUGGCCGAUGCUAAGAACAUUAAGAAGGGCCCUGCUCCCUUCUACCCUCUGGAG
GAUGGCACCGCUGGCGAGCAGCUGCACAAGGCCAUGAAGAGGUAUGCCCUGGUGC
CUGGCACCAUUGCCUUCACCGAUGCCCACAUUGAGGUGGACAUCACCUAUGCCGA
GUACUUCGAGAUGUCUGUGCGCCUGGCCGAGGCCAUGAAGAGGUACGGCCUGAAC
ACCAACCACCGCAUCGUGGUGUGCUCUGAGAACUCUCUGCAGUUCUUCAUGCCAG
UGCUGGGCGCCCUGUUCAUCGGAGUGGCCGUGGCCCCUGCUAACGACAUUUACAA
CGAGCGCGAGCUGCUGAACAGCAUGGGCAUUUCUCAGCCUACCGUGGUGUUCGUG
UCUAAGAAGGGCCUGCAGAAGAUCCUGAACGUGCAGAAGAAGCUGCCUAUCAUCC
AGAAGAUCAUCAUCAUGGACUCUAAGACCGACUACCAGGGCUUCCAGAGCAUGUA
CACAUUCGUGACAUCUCAUCUGCCUCCUGGCUUCAACGAGUACGACUUCGUGCCA
GAGUCUUUCGACAGGGACAAAACCAUUGCCCUGAUCAUGAACAGCUCUGGGUCUA
CCGGCCUGCCUAAGGGCGUGGCCCUGCCUCAUCGCACCGCCUGUGUGCGCUUCUC
UCACGCCCGCGACCCUAUUUUCGGCAACCAGAUCAUCCCCGACACCGCUAUUCUG
AGCGUGGUGCCAUUCCACCACGGCUUCGGCAUGUUCACCACCCUGGGCUACCUGA
UUUGCGGCUUUCGGGUGGUGCUGAUGUACCGCUUCGAGGAGGAGCUGUUCCUGC
GCAGCCUGCAAGACUACAAAAUUCAGUCUGCCCUGCUGGUGCCAACCCUGUUCAG
CUUCUUCGCUAAGAGCACCCUGAUCGACAAGUACGACCUGUCUAACCUGCACGAG
AUUGCCUCUGGCGGCGCCCCACUGUCUAAGGAGGUGGGCGAAGCCGUGGCCAAGC
GCUUUCAUCUGCCAGGCAUCCGCCAGGGCUACGGCCUGACCGAGACAACCAGCGC
CAUUCUGAUUACCCCAGAGGGCGACGACAAGCCUGGCGCCGUGGGCAAGGUGGUG
CCAUUCUUCGAGGCCAAGGUGGUGGACCUGGACACCGGCAAGACCCUGGGAGUGA
ACCAGCGCGGCGAGCUGUGUGUGCGCGGCCCUAUGAUUAUGUCCGGCUACGUGAA
UAACCCUGAGGCCACAAACGCCCUGAUCGACAAGGACGGCUGGCUGCACUCUGGC
GACAUUGCCUACUGGGACGAGGACGAGCACUUCUUCAUCGUGGACCGCCUGAAGU

CUCUGAUCAAGUACAAGGGCUACCAGGUGGCCCCAGCCGAGCUGGAGUCUAUCCU
GCUGCAGCACCCUAACAUUUCGACGCCGGAGUGGCCGGCCUGCCCGACGACGAU
GCCGGCGAGCUGCCUGCCGCCGUCGUCGUGCUGGAACACGGCAAGACCAUGACCG
AGAAGGAGAUCGUGGACUAUGUGGCCAGCCAGGUGACAACCGCCAAGAAGCUGC
GCGGCGGAGUGGUGUUCGUGGACGAGGUGCCCAAGGGCCUGACCGGCAAGCUGG
ACGCCCGCAAGAUCCGCGAGAUCCUGAUCAAGGCUAAGAAAGGCGGCAAGAUCGC
CGUGUAAGGAAGGGGGAAGGAAAGGGA

The above is only preferred embodiments of the present disclosure, and is not intended to limit the present disclosure in any form. Under the teaching of the above-disclosed technical contents, simple modifications or equivalent changes by those skilled in the art fall within the scope of protection of the present disclosure.

## Claims

1. A miRNA-2911 and the nucleic acid molecule derived therefrom for improving the stability of a nucleic acid of interest, **characterized in that** the nucleotide sequence of the miRNA-2911 and the nucleic acid molecule derived therefrom comprises the following nucleic acid sequence:
GGX₁X₂GGGGG-(L)ₙ-X₃X₄GGX₅X₆X₇GGGX₈ (SEQ ID NO: 1)
wherein:
X₁, X₂, X₃, X₄, X₅, X₆, X₇ and X₈ are independently selected from A, C, T and U;
n is 0 or 1;
L represents a linker group of no more than 10 nucleotides in length.

2. The miRNA-2911 and the nucleic acid molecule derived therefrom according to claim 1, **characterized in that** L represents GGGGG (SEQ ID NO: 30).

3. The miRNA-2911 and the nucleic acid molecule derived therefrom according to claim 1, **characterized in that** the miRNA-2911 and the nucleic acid molecule derived therefrom comprise the following nucleic acid sequence:
GGX₁X₂GGGGGX₃X₄GGX₅X₆X₇GGGX₈ (SEQ ID NO: 3)
wherein:
X₁, X₂, X₃, X₄, X₅, X₆, X₇ and X₈ are independently selected from A, C, T and U.

4. The miRNA-2911 and the nucleic acid molecule derived therefrom according to any one of claims 1-3, **characterized in that**:
X₁, X₂, X₄ and X₆ are identical, optionally are A, C or U; and/or
X₃, X₅ and X₈ are identical, optionally are A, C or U.

5. The miRNA-2911 and the nucleic acid molecule derived therefrom according to any one of claims 1-4, **characterized in that** the sequence of the miRNA-2911 and the nucleic acid molecule derived therefrom is as shown in any one of SEQ ID NO: 2, and SEQ ID NO: 4-10.

6. A protected nucleic acid of interest, **characterized in that**:
the miRNA-2911 and the nucleic acid molecule derived therefrom according to any one of claims 1-5 are conjugated to the 5' and/or 3' end of the nucleic acid of interest, or
the miRNA-2911 and the nucleic acid molecule derived therefrom according to any one of claims 1-5 are inserted into the nucleic acid of interest;
and, the stability of the protected nucleic acid of interest is improved.

7. The protected nucleic acid of interest according to claim 6, **characterized in that**:
the nucleic acid of interest is ≥ 8 nt in length , preferably 8-5000 nt;
preferably, the nucleic acid of interest is a nucleic acid for gene knockdown, a nucleic acid for gene knockout, a nucleic acid for gene activation, a nucleic acid for gene modification, a nucleic acid for gene editing, a nucleic acid for gene regulation, a nucleic acid for protein regulation, a nucleic acid for protein expression, a nucleic acid for bioassay or a nucleic acid drug;
more preferably, the nucleic acid of interest is:
(1) an oligonucleotide, including but not limited to an antisense oligonucleotide ASO or an apatmer;
(2) a single-stranded DNA, a double-stranded DNA, a single-stranded RNA, a double-stranded RNA, an mRNA or an ncRNA; the ncRNA includes but not limited to miRNA, siRNA, saRNA, piRNA, lncRNA, circRNA, fragments thereof, or other regulatory RNA.

8. A pharmaceutical composition, **characterized in that** the pharmaceutical composition comprises:
(1) a therapeutically effective amount of the protected nucleic acid of interest according to any one of claims 6-7, and
(2) a pharmaceutically acceptable carrier.

9. A method for improving the stability of a nucleic acid of interest, **characterized in that**:
the miRNA-2911 and the nucleic acid molecule derived therefrom according to any one of claims 1-5 are conjugated to the 5' end and/or 3' end of the nucleic acid of interest, or inserted into the nucleic acid of interest.

10. A method according to claim 9, **characterized in that**:
the nucleic acid of interest is ≥ 8 nt in length, preferably 8-5000 nt;
preferably, the nucleic acid of interest is a nucleic acid for gene knockdown, a nucleic acid for gene knockout, a nucleic acid for gene activation, a nucleic acid for gene modification, a nucleic acid for gene editing, a nucleic acid for gene regulation, a nucleic acid for protein regulation, a nucleic acid for protein expression, a nucleic acid for bioassay or a nucleic acid drug;
more preferably, the nucleic acid of interest is:
(1) an oligonucleotide, including but not limited to an antisense oligonucleotide ASO or an apatmer;
(2) a single-stranded DNA, a double-stranded DNA, a single-stranded RNA, a double-stranded RNA, an mRNA or an ncRNA; the ncRNA includes but not limited to miRNA, siRNA, saRNA, piRNA, lncRNA, circRNA, fragments thereof, or other regulatory RNA.
